(19) Europäisches Patentamt
European Patent Office
Office européen des brevets

(11) EP 4 796 117 A1

(12) EUROPEAN PATENT APPLICATION
published in accordance with Art. 153(4) EPC

(43) Date of publication:
26.08.2026 Bulletin 2026/35

(21) Application number: 24896538.6

(22) Date of filing: 26.11.2024

(51) International Patent Classification (IPC):
*A61K 31/728* (2006.01) *A61P 31/00* (2006.01)

(86) International application number:
PCT/CN2024/134646

(87) International publication number:
WO 2025/113451 (05.06.2025 Gazette 2025/23)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH MA MD TN

(30) Priority: 30.11.2023 CN 202311634295
18.01.2024 CN 202410080031

(71) Applicant: Bloomage Biotechnology Corporation Limited
Jinan, Shandong 250101 (CN)

(72) Inventors:
- LIU, Yijie
Jinan, Shandong 250101 (CN)
- WU, Yue
Jinan, Shandong 250101 (CN)

(74) Representative: Huang, Liwei
Cäcilienstraße 12
40597 Düsseldorf (DE)

# (54) USE OF HYALURONIC ACID OR SALT THEREOF IN PREPARING ANTIMICROBIAL PEPTIDE INDUCER

(57) The present invention provides use of hyaluronic acid or a salt thereof in the preparation of an antimicrobial peptide inducer, and relates to the technical field of antimicrobial care. The reducing end of the hyaluronic acid or the salt thereof comprises a uronic acid group. It has been discovered for the first time that hyaluronic acid with a specific terminal structure has the effect of inducing the generation of an antimicrobial peptide. This discovery provides a new direction for elucidating the correlation between the structure and efficacy of the hyaluronic acid, and also offers a new effective ingredient for regulating cellular basal defense to inhibit pathogenic bacterial invasion.

HBD2
Relative fluorescence intensity (%)
400
300
200
100
0
**
**
ns
ns
Control group
Test group 1
Test group 2
Test group 3
Test group 4

FIG. 1



Processed by Luminess, 75001 PARIS (FR)

## Description

### CROSS-REFERENCE TO RELATED APPLICATION

This application claims the priority of Chinese Patent Application No. 202311634295.3 filed with the China National Intellectual Property Administration on November 30, 2023 and entitled "USE OF HYALURONIC ACID OR SALT THEREOF IN PREPARATION OF ANTIMICROBIAL PEPTIDE INDUCER", the disclosure of which is incorporated by reference herein in its entirety as part of the present invention; and

This application claims the priority of Chinese Patent Application No. 202410080031.6 filed with the China National Intellectual Property Administration on January 18, 2024 and entitled "USE OF HYALURONIC ACID OR SALT THEREOF IN PREPARATION OF ANTIMICROBIAL PEPTIDE INDUCER", the disclosure of which is incorporated by reference herein in its entirety as part of the present invention.

### TECHNICAL FIELD

The present invention relates to the technical field of antimicrobial care, in particular to use of hyaluronic acid or a salt thereof in the preparation of an antimicrobial peptide inducer.

### BACKGROUND

In daily life, people are exposed to potential hazards from the external environment, such as pathogenic microorganisms, chemical agents and allergens in the environment, which can infect the skin, oral cavity, intestines, urinary tract, respiratory tract and the like of the human. Thus, regulating cellular basal defense to inhibit pathogenic bacterial invasion is of important significance.

An antimicrobial peptide, also known as an antimicrobic peptide and polypeptide antibiotics, is a class of small molecule peptides with broad-spectrum resistance, which is an important component for innate immunity of organisms. In the skin, keratinocytes can secrete the antimicrobial peptide to resist pathogen invasion, thereby exerting the effects of killing microorganisms and regulating microecological balance. There are many types of antimicrobial peptides. Among them, human β-defensin 2 (HBD2) is a major inducible peptide, which plays a crucial role in host defense.

### SUMMARY

Through research, the inventors of the present invention have unexpectedly found that hyaluronic acid, when possessing a specific terminal structure, exhibits the effect of inducing the generation of human β-defensin 2 (HBD2), thereby completing the present invention.

In one aspect, the present invention provides use of hyaluronic acid or a salt thereof in the preparation of an antimicrobial peptide inducer, wherein the reducing end of the hyaluronic acid or the salt thereof comprises a uronic acid group.

In another aspect, the present invention provides use of hyaluronic acid or a salt thereof as an antimicrobial peptide inducer, wherein the reducing end of the hyaluronic acid or the salt thereof comprises a uronic acid group.

In still another aspect, the present invention provides use of hyaluronic acid or a salt thereof in the preparation of a product for promoting the expression of an antimicrobial peptide, wherein the reducing end of the hyaluronic acid or the salt thereof comprises a glucuronic acid group.

Optionally, the molecular weight of the hyaluronic acid or the salt thereof is less than 5000 Da.

More optionally, the molecular weight of the hyaluronic acid or the salt thereof is less than 5000 Da and greater than 300 Da.

More optionally, the molecular weight of the hyaluronic acid or the salt thereof is 800-4999 Da.

More optionally, the molecular weight of the hyaluronic acid or the salt thereof is 800-1000 Da.

It can be understood that the molecular weight of the hyaluronic acid or the salt thereof can be any one selected from the group consisting of 300 Da, 400 Da, 500 Da, 600 Da, 700 Da, 800 Da, 900 Da, 1100 Da, 1200 Da, 1300 Da, 1400 Da, 1500 Da, 2000 Da, 2500 Da, 3000 Da, 3500 Da, 4000 Da, 4500 Da, 4600 Da, 4700 Da, 4800 Da, 4900 Da and 4999 Da.

Further, the structural formula of the hyaluronic acid or the salt thereof is represented by Formula (I):

Formula (I)

wherein, X is selected from H, K, Na, Ca or Zn, and n is any integer selected from 0 to 5.

Optionally, the X is Na.

The n is any integer selected from 0 to 5. n is any integer selected from the group consisting of 0, 1, 2, 3, 4 and 5.

Optionally, based on mass concentration percentage, the use concentration of the hyaluronic acid or the salt thereof is 0.0001%-5%; more optionally, the use concentration of the hyaluronic acid or the salt thereof is 0.01%-1%.

The use concentration of the hyaluronic acid or the salt thereof can be any one selected from the group consisting of 0.0001%, 0.0005%, 0.001%, 0.005%, 0.01 %, 0.02 %, 0.03 %, 0.04%, 0.05%, 0.06%, 0.07%, 0.08%, 0.09%, 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5% and 5%.

In an optional embodiment, the hyaluronic acid or the salt thereof is sodium hyaluronate, which is purchased from Bloomage Biotechnology Corporation Limited under the trade name Weizhen.

Further, the antimicrobial peptide comprises human β-defensin 2 (HBD2).

In the present invention, it is found for the first time that the hyaluronic acid or the salt thereof having the uronic acid group at the reducing end can be used as the antimicrobial peptide inducer to promote the generation of the antimicrobial peptide in cells, thereby inhibiting the growth and proliferation of harmful microbes in the human body to exert the effect of regulating the human microecology, and improving the cellular basal defense capacity to achieve the purpose of inhibiting pathogenic bacterial invasion.

In an optional embodiment, the cell comprises epithelial cells and mucosal cells.

In an optional embodiment, the epithelial cells comprise cutaneous epithelial cells, genital tract epithelial cells, intestinal epithelial cells, oral epithelial cells, urinary tract epithelial cells and keratinocytes.

In an optional embodiment, the epithelial cells comprise keratinocytes, vaginal epithelial cells and intestinal epithelial cells.

In an optional embodiment, the mucosal cells comprise mucocutaneous cells, genital tract mucosal cells, intestinal mucosal cells, oral mucosal cells and urinary tract mucosal cells.

In an optional embodiment, the mucosal cells comprise oral mucosal cells.

In yet another aspect, the present invention further provides use of the aforementioned hyaluronic acid or a salt thereof in promoting the generation of an antimicrobial peptide in cells.

In a further aspect, the present invention provides an antimicrobial peptide inducer, the antimicrobial peptide inducer comprising the aforementioned hyaluronic acid or the salt thereof.

In a still further aspect, the present invention further provides use of the aforementioned hyaluronic acid or the salt thereof in regulating human microecology, wherein the reducing end of the hyaluronic acid or the salt thereof comprises a uronic acid group.

The human microecology comprises oral microecology, urinary tract microecology, intestinal microecology, genital tract microecology and skin microecology.

Further, regulating human microecology comprises inhibiting the growth and proliferation of harmful microbes in the human body.

Further, inhibiting the growth and proliferation of harmful microbes in the human body comprises promoting the generation of the antimicrobial peptide in cells.

Further, the harmful microbes comprise harmful bacteria. The harmful bacteria comprise Gram-positive bacteria and/or Gram-negative bacteria.

Further, the Gram-positive bacteria comprise *Staphylococcus* bacteria.

Further, the *Staphylococcus* bacteria comprise *Staphylococcus aureus*.

Optionally, the inhibition rate of the hyaluronic acid or the salt thereof against *Staphylococcus aureus* is 97%.

Further, the Gram-negative bacteria comprise *Pseudomonas* bacteria.

Further, the *Pseudomonas* bacteria comprise *Pseudomonas aeruginosa*.

Optionally, the inhibition rate of the hyaluronic acid or the salt thereof against *Pseudomonas aeruginosa* is 99%.

Further, the Gram-negative bacteria comprise *Escherichia* bacteria.
Further, the *Escherichia* bacteria comprise *Escherichia coli*.
Optionally, the inhibition rate of the hyaluronic acid or the salt thereof against *Escherichia coli* is 97%.
In another aspect, the present invention further provides use of the aforementioned hyaluronic acid or a salt thereof in improving cellular defense capacity.
Further, the cellular defense capacity is cellular basal defense capacity.
The present invention has the following benefits:
In the present invention, it is found for the first time that the hyaluronic acid with the specific terminal structure exhibits the effect of inducing the generation of the antimicrobial peptide, which sheds new light on the correlation between the structure and efficacy of the hyaluronic acid, and also provides a novel active ingredient for modulating cellular basal defense to inhibit the pathogenic bacterial invasion.

## BRIEF DESCRIPTION OF THE DRAWINGS

The accompanying drawings described herein are provided for a further understanding of the present invention and constitute a part thereof. The exemplary embodiments of the present invention and the descriptions thereof are used to interpret the present invention and do not constitute an improper limitation thereof. In the figures:

FIG. 1 shows the relative fluorescence quantitative results of HBD2 expression in keratinocytes;
FIG. 2 shows the relative fluorescence quantitative results of HBD2 expression in vaginal cells;
FIG. 3 shows the relative fluorescence quantitative results of HBD2 expression in intestinal cells; and
FIG. 4 shows the relative fluorescence quantitative results of HBD2 expression in oral cells.

## DETAILED DESCRIPTION OF THE EMBODIMENTS

To more clearly illustrate the overall concept of the present invention, detailed description will be made in combination with the drawings of the specification by means of embodiments. In the following description, numerous specific details are set forth in order to provide a thorough understanding of the present invention. However, it will be apparent to those skilled in the art that the present invention may be practiced without one or more of these details. In other examples, certain technical features well known in the art have not been described to avoid obscuring the present invention.
If no specific conditions are indicated in the examples, the experiments shall be carried out according to conventional conditions or conditions recommended by the manufacturer.
Sodium hyaluronate-1 and sodium hyaluronate-2 are purchased from Bloomage Biotech; DMEM medium is purchased from Gibco; *Staphylococcus aureus* ATCC29213, *Pseudomonas aeruginosa* ATCC9027 and pathogenic *Escherichia coli* ATCC8739 are purchased from the American Type Culture Collection (ATCC). Detailed commercial information is shown in Table 1.
Unless otherwise specified, in the following embodiments, reagents or instruments without indicated manufacturers are conventional products commercially available.
Unless otherwise stated, the experimental methods, detection methods and preparation methods disclosed in the present invention all employ conventional techniques of molecular biology, biochemistry, chromatin structure and analysis, analytical chemistry, cell culture, recombinant DNA technology and conventional techniques in the relevant fields, which may be carried out in accordance with Molecular Cloning: A Laboratory Manual (Fourth Edition).
It is noted that in the specification and claims, certain terms are used to refer to specific components. Those skilled in the art should understand that the same component may be referred to by different terms. The specification and claims do not distinguish components based on differences in terminology, but rather on functional differences as the criterion for distinction. As used throughout the specification and claims, the terms "comprise" or "include" are open-ended terms and shall therefore be construed to mean "including but not limited to". The subsequent descriptions in the specification set forth preferred embodiments for carrying out the present invention; however, such descriptions are intended for the purpose of general principles of the specification and are not intended to limit the scope of the present invention. The scope of protection of the present invention shall be defined by the appended claims.

Table 1

| | Source |
|---|---|
| Sodium hyaluronate-1 | Bloomage Biotechnology Corporation Limited, trade name: Weizhen, LOT: 20201012-1, molecular weight: 800-1000 Da |

(continued)

| | Source |
|---|---|
| Sodium hyaluronate-2 | Bloomage Biotechnology Corporation Limited, LOT: 20201216, molecular weight: 800-1000 Da |

Example 1 Identification of reducing end of hydrolyzed hyaluronic acid

Experimental principle: the reducing end of sodium hyaluronate is determined by colorimetry using Morgan-Elson reaction. If the color of the solution turns red, the reducing end is N-acetylglucosamine; and if no color changes, the reducing end is glucuronic acid.

Reaction buffer: an alkaline borate solution (1.73 g of $H_3BO_3$ and 0.78 g of KOH were dissolved in 10 mL of water, and one-tenth of its volume of 0.8 g/mL $K_2CO_3$ solution was added before use); and a p-dimethylaminobenzaldehyde solution (2 g of p-dimethylaminobenzaldehyde is dissolved in 2.5 mL of concentrated hydrochloric acid and 7.5 mL of glacial acetic acid, and then the resulting solution was diluted with 4 volumes of glacial acetic acid before use).

Experimental steps: 400 $\mu$L of 10 g/L sodium hyaluronate (sodium hyaluronate-1 and sodium hyaluronate-2) solution was taken, 110 $\mu$L of alkaline borate solution was added to the above solution, and then the mixture was boiled for 4 min; 1.5 mL of p-dimethylaminobenzaldehyde solution was added to the boiled mixture; and the resulting mixture was then incubated at 37°C for 20 min.

The results are as shown in Table 2. Upon Morgan-Elson reaction, sodium hyaluronate-1 does not show red, indicating that the reducing end is uronic acid; and upon Morgan-Elson reaction, sodium hyaluronate-2 shows red, indicating that the reducing end is N-acetylglucosamine.

Table 2

| | Morgan-Elson reaction results |
|---|---|
| Sodium hyaluronate-1 | No redness |
| Sodium hyaluronate-2 | Redness |

Example 2 Antimicrobial peptide HBD2 expression assay

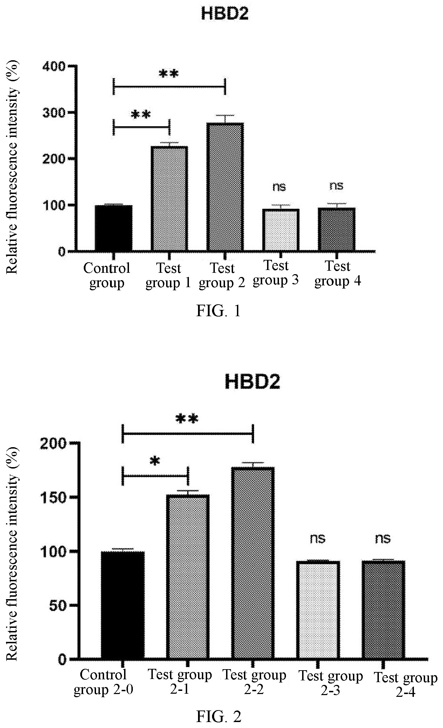

Sodium hyaluronate-1 and Sodium hyaluronate-2 were prepared into different experimental sample solutions using DMEM medium. Their effects on the expression of the antimicrobial peptide HBD2 in primary keratinocytes and human vaginal epithelial cells were determined. A specific method is as follows: primary keratinocytes (epidermal keratinocytes (HEKn) were isolated from neonatal foreskin in the laboratory and cryopreserved at the end of primary culture) and human vaginal epithelial cells (VK2/E6E7, purchased from ATCC® CRL-2616™) were respectively co-cultured with different experimental sample solutions for 1 day; then, the experimental sample solutions were removed, and the cells were fixed with methanol, followed by blocking with a blocking solution for 1 h; after washing with PBS, an HBD2 primary antibody was added, and the cells were incubated overnight at 4°C; subsequently, a diluted secondary antibody was added for staining for 1.5 h; after mounting with an antifade mounting medium, the cells were observed and photographed under a fluorescence microscope; and then the relative fluorescence intensity was statistically analyzed by semi-quantitative fluorescence analysis using ImageJ. The results of the keratinocyte experimental group are shown in FIG. 1 and Table 3. The results of the vaginal epithelial cell experimental group are shown in FIG. 2 and Table 4.

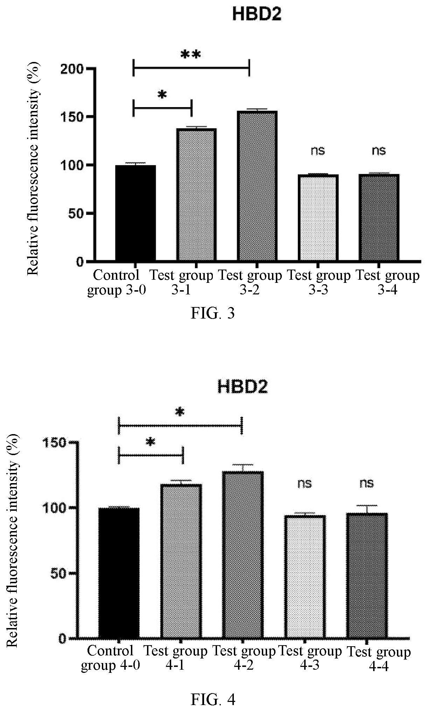

Sodium hyaluronate-1 and Sodium hyaluronate-2 were prepared into different experimental sample solutions using McCoy's 5A medium (Procell, #PM150710). Their effects on the expression of antimicrobial peptide HBD2 in human intestinal epithelial cells were determined. A specific method is as follows: human colon cancer cells HT29 were selected as commonly used cells for in vitro studies of intestinal epithelial cells; the intestinal epithelial cells (HT29, purchased from Procell, #CL-0118) were co-cultured with different experimental sample solutions for 1 day; subsequently, the experimental sample solutions were removed, and the cells were fixed with methanol, followed by blocking with a blocking solution for 1 h; after washing with PBS, an HBD2 primary antibody was added, and the cells were incubated overnight at 4°C; a diluted secondary antibody was then added for staining for 1.5 h; after mounting with an antifade mounting medium, the cells were observed and photographed under a fluorescence microscope; and then the relative fluorescence intensity was statistically analyzed by semi-quantitative fluorescence analysis using ImageJ. The results of the intestinal epithelial cell experimental group are shown in FIG. 3 and Table 5.

Sodium hyaluronate-1 and Sodium hyaluronate-2 were prepared into different experimental sample solutions using a medium (Procell, #SNPM-H151). Their effects on the expression of antimicrobial peptide HBD2 in human oral mucosal cells were determined. A specific method is as follows: human oral mucosal cells were selected as commonly

used cells SNP-H151 for mucosal in vitro studies; the oral mucosal cells (SNP-H151, purchased from Procell, #SNPM-H151) were co-cultured with different experimental sample solutions for 1 day; subsequently, the experimental sample solutions were removed, and the cells were fixed with methanol, followed by blocking with a blocking solution for 1 h; after washing with PBS, an HBD2 primary antibody was added, and the cells were incubated overnight at 4°C; a diluted secondary antibody was then added for staining for 1.5 h; after mounting with an antifade mounting medium, the cells were observed and photographed under a fluorescence microscope; and then the relative fluorescence intensity was statistically analyzed by semi-quantitative fluorescence analysis using ImageJ. The results are as shown in FIG. 4 and Table 6.

On the basis of satisfying the requirement of significant difference, the stronger relative fluorescence intensity indicates the better cellular self-defense effect.

Table 3 Expression results of antimicrobial peptide HBD2 in keratinocytes

| Group | Treatment method | Relative fluorescence intensity % | Significance |
|---|---|---|---|
| Control group | DMEM medium | 100.00 | - |
| Test group 1 | 0.1% (w/v) sodium hyaluronate-1 | 227.84 | ** |
| Test group 2 | 0.2% (w/v) sodium hyaluronate-1 | 279.49 | ** |
| Test group 3 | 0.1% (w/v) sodium hyaluronate-2 | 92.31 | ns |
| Test group 4 | 0.2% (w/v) sodium hyaluronate-2 | 94.26 | ns |

Table 4 Expression results of antimicrobial peptide HBD2 in vaginal epithelial cells

| Group | Treatment method | Relative fluorescence intensity % | Significance |
|---|---|---|---|
| Control group 2-0 | DMEM medium | 100.00 | - |
| Test group 2-1 | 0.1% (w/v) sodium hyaluronate-1 | 152.33 | * |
| Test group 2-2 | 0.2% (w/v) sodium hyaluronate-1 | 178.92 | ** |
| Test group 2-3 | 0.1% (w/v) sodium hyaluronate-2 | 91.21 | ns |
| Test group 2-4 | 0.2% (w/v) sodium hyaluronate-2 | 91.55 | ns |

Table 5 Expression results of antimicrobial peptide HBD2 in intestinal epithelial cells

| Group | Treatment method | Relative fluorescence intensity % | Significance |
|---|---|---|---|
| Control group 3-0 | McCoy's 5A medium | 100.00 | - |
| Test group 3-1 | 0.1% (w/v) sodium hyaluronate-1 | 138.53 | * |
| Test group 3-2 | 0.2% (w/v) sodium hyaluronate-1 | 156.58 | ** |
| Test group 3-3 | 0.1% (w/v) sodium hyaluronate-2 | 90.66 | ns |
| Test group 3-4 | 0.2% (w/v) sodium hyaluronate-2 | 91.23 | ns |

Table 6 Expression results of antimicrobial peptide HBD2 in oral mucosal cells

| Group | Treatment method | Relative fluorescence intensity % | Significance |
|---|---|---|---|
| Control group 4-0 | Medium | 100.00 | - |
| Test group 4-1 | 0.1% (w/v) sodium hyaluronate-1 | 118.24 | * |
| Test group 4-2 | 0.2% (w/v) sodium hyaluronate-1 | 136.28 | * |
| Test group 4-3 | 0.1% (w/v) sodium hyaluronate-2 | 95.00 | ns |
| Test group 4-4 | 0.2% (w/v) sodium hyaluronate-2 | 98.52 | ns |

As can be seen from Tables 3-6 and FIGs.1-4, sodium hyaluronate-1 significantly promotes the expression of the antimicrobial peptide HBD2 when administered to keratinocytes, vaginal epithelial cells, intestinal epithelial cells and oral mucosal cells, whereas sodium hyaluronate-2 exhibits no such promoting effect. That is to say, the hyaluronic acid with the

uronic acid terminus is capable of promoting the expression of antimicrobial peptide HBD2 in cells.

Example 3 Antibacterial efficacy test

1. Sample preparation

1.1 Sample solutions in Test groups

Sodium hyaluronate-1 and sodium hyaluronate-2 were separately diluted with DMEM medium (Gibco #100569010) to a concentration of 0.5% (w/v) to obtain a sample solution of Test group A and a sample solution of Test group B, respectively.

Keratinocytes (human epidermal keratinocytes (HEKn) were isolated from neonatal foreskin in the laboratory and cryopreserved at the end of primary culture) were co-cultured with sample solutions of Test groups A and B at 37°C under 5% $CO_2$ for 1 day, respectively. After the solutions were removed, the cells were washed with PBS and lysed with Roswell Park Memorial Institute (RPMI) lysis buffer (Solarbio). The resulting mixtures were centrifuged to collect the supernatants as Test group C and Test group D, respectively.

Vaginal epithelial cells (VK2/E6E7, purchased from ATCC#CRL-2616) were co-cultured with sample solutions from Test group A and Test group B at 37°C under 5% $CO_2$ for 1 day, respectively. After the solutions were removed, the cells were washed with PBS and lysed with RPMI lysis buffer (Solarbio). The resulting mixtures were centrifuged to collect the supernatants as Test group E and Test group F, respectively.

Sodium hyaluronate-1 and sodium hyaluronate-2 were separately diluted with McCoy's 5A medium (Procell, #PM150710) to a concentration of 0.5% (w/v) to obtain a sample solution of Test group a and a sample solution of Test group b, respectively.

Intestinal epithelial cells (HT29, purchased from Procell, #CL-0118) were co-cultured with sample solutions from Test group a and Test group b at 37°C under 5% $CO_2$ for 1 day, respectively. After the solutions were removed, the cells were washed with PBS and lysed with RPMI lysis buffer (Solarbio). The resulting mixtures were centrifuged to collect the supernatants as Test group G and Test group H, respectively.

Sodium hyaluronate-1 and sodium hyaluronate-2 were separately diluted with a medium (Procell, #SNPM-H151) to a concentration of 0.5% (w/v) to obtain a sample solution of Test group a' and a sample solution of Test group b', respectively.

Human oral mucosal cells (SNP-H151, purchased from Procell, #SNPM-H151) were co-cultured with sample solutions from Test groups a' and b' respectively at 37°C under 5% $CO_2$ for 1 day. After the solutions were removed, the cells were washed with PBS and lysed with RPMI lysis buffer (Solarbio). The resulting mixtures were centrifuged to collect supernatants as Test group I and Test group J, respectively.

1.2 Sample solution in control group

Sterile PBS was used as control group.

2. Preparation of bacterial suspension

*Staphylococcus aureus* ATCC29213 was cultured in nutrient broth with shaking at 37°C for 48 h, *Pseudomonas aeruginosa* ATCC9027 was cultured in nutrient broth with shaking at 37°C for 18 h, and pathogenic *Escherichia coli* ATCC8739 was cultured in nutrient broth with shaking at 37°C for 48 h. Each strain was then diluted with sterile PBS to prepare a bacterial suspension of $1\times10^5$-$9\times10^5$ CFU/mL.

3. Detection of antibacterial effects against *Staphylococcus aureus*, *Pseudomonas aeruginosa* and pathogenic *Escherichia coli*

The control group, Test group A, Test group B, Test group C and Test group D were respectively mixed thoroughly with suspensions of *Staphylococcus aureus* and *Pseudomonas aeruginosa* in a ratio of 1000 μL: 100 μL (a total volume of 3 mL) to obtain mixed solutions. The mixed solutions in each test group and the control group were simultaneously incubated in an incubator at 37°C for 3 h. 500 μL of each mixed solution was separately aspirated, and then diluted to 1/10 and 1/100 sample solutions with sterile PBS.100 μL of each sample solution was aspirated separately, spread on TSA agar (Tryptic Soy Agar) plates, and incubated at 32.5°C for 41 h. Plate counting was performed, with three parallel plates prepared for each sample solution. The mean values of the three parallel plates were calculated to determine the inhibition rate. The detailed antibacterial results are shown in Table 7.

The control group, Test group A, Test group B, Test group a, Test group b, Test group E, Test group F, Test group G

and Test group H were evenly mixed with a pathogenic *Escherichia coli* suspension in a ratio of 1000 μL: 100 μL (a total volume was 3 mL) respectively to obtain mixed solutions. The mixed solutions in each test group and the control group were simultaneously incubated in an incubator at 37°C for 3 h.500 μL of each mixed solution was separately aspirated, and then diluted to 1/10 and 1/100 sample solutions with sterile PBS.100 μL of each sample solution was aspirated separately, spread on TSA agar (Tryptic Soy Agar) plates, and incubated at 32.5°C for 41 h. Plate counting was performed, with three parallel plates prepared for each sample solution. The mean values of the three parallel plates were calculated to determine the inhibition rate. The detailed antibacterial results are shown in Table 8 and Table 9.

The control group, Test group a', Test group b', Test group I and Test group J were evenly mixed with a *Staphylococcus aureus* suspension in a ratio of 1000 μL: 100 μL (a total volume was 3 mL) respectively to obtain mixed solutions. The mixed solutions in each test group and the control group were simultaneously incubated in an incubator at 37°C for 3 h.500 μL of each mixed solution was separately aspirated, and then diluted to 1/10 and 1/100 sample solutions with sterile PBS. 100 μL of each sample solution was aspirated separately, spread on TSA agar (Tryptic Soy Agar) plates, and incubated at 32.5°C for 41 h. Plate counting was performed, with three parallel plates prepared for each sample solution. The mean values of the three parallel plates were calculated to determine the inhibition rate. The detailed antibacterial results are shown in Table 10.

Inhibition rate (%) = (Average value of Control group - Average value of Test group) / Average value of Control group × 100%

The evaluation was performed in accordance with Evaluation Method for Antimicrobial and Antibacterial Effects (WS/T 650-2019) issued by the National Health Commission of the People's Republic of China. The inhibition rate of 50%-90% indicates antibacterial activity; the inhibition rate of ≥90% indicates strong antibacterial activity; and the rate of less than 50% is regarded as no antibacterial activity.

Table 7 Antibacterial results of keratinocyte lysate treatment group

| Group | Treatment method | Inhibition rate against *Staphylococcus aureus* | Inhibition rate against *Pseudomonas aeruginosa* |
|---|---|---|---|
| Control group | Sterile PBS | 10% | 8% |
| Test group A | 0.5% sodium hyaluronate-1 | 13% | 11% |
| Test group B | 0.5% sodium hyaluronate-2 | 25% | 21% |
| Test group C | Cell lysate after treatment with sodium hyaluronate-1 | 97% | 99% |
| Test group D | Cell lysate after treatment with sodium hyaluronate-2 | 30% | 25% |

Table 8 Antibacterial results of vaginal epithelial cell lysate treatment group

| Group | Treatment method | Inhibition rate against pathogenic *Escherichia coli* |
|---|---|---|
| Control group | Sterile PBS | 11% |
| Test group A | 0.5% sodium hyaluronate-1 | 15% |
| Test group B | 0.5% sodium hyaluronate-2 | 22% |
| Test group E | Cell lysate after treatment with sodium hyaluronate-1 | 99% |
| Test group F | Cell lysate after treatment with sodium hyaluronate-2 | 29% |

Table 9 Antibacterial results of intestinal epithelial cell lysate treatment group

| Group | Treatment method | Inhibition rate against pathogenic *Escherichia coli* |
|---|---|---|
| Control group | Sterile PBS | 11% |
| Test group a | 0.5% sodium hyaluronate-1 | 15% |
| Test group b | 0.5% sodium hyaluronate-2 | 22% |

(continued)

| Group | Treatment method | Inhibition rate against pathogenic *Escherichia coli* |
|---|---|---|
| Test group G | Cell lysate after treatment with sodium hyaluronate-1 | 98% |
| Test group H | Cell lysate after treatment with sodium hyaluronate-2 | 36% |

Table 10 Antibacterial results of oral mucosal cell lysate treatment group

| Group | Treatment method | Inhibition rate against *Staphylococcus aureus* |
|---|---|---|
| Control group | Sterile PBS | 11% |
| Test group a' | 0.5% sodium hyaluronate-1 | 18% |
| Test group b' | 0.5% sodium hyaluronate-2 | 20% |
| Test group I | Cell lysate after treatment with sodium hyaluronate-1 | 97% |
| Test group J | Cell lysate after treatment with sodium hyaluronate-2 | 30% |

As can be seen from Table 7, the initial concentration of the *Staphylococcus aureus* suspension is $2.8\times10^5$ CFU/mL, the inhibition rate for the control group is 10%, while the bacterial inhibition rate of the *Staphylococcus aureus* for the cell lysate group treated with sodium hyaluronate-1 is 97%. The results indicate that the cell lysate treated with sodium hyaluronate-1 can inhibit the growth of *Staphylococcus aureus*, whereas the cell lysate treated with sodium hyaluronate-2 exhibits no significant antibacterial effect. In addition, the initial concentration of the *Pseudomonas aeruginosa* suspension is $2.9\times10^5$ CFU/mL. As shown in Table 7, the inhibition rate for the control group is 8%, while the inhibition rate of *Pseudomonas aeruginosa* for the keratinocyte lysate group treated with sodium hyaluronate-1 is 99%. The results indicate that the keratinocyte lysate treated with sodium hyaluronate-1 can inhibit the growth of *Pseudomonas aeruginosa*, whereas the keratinocyte lysate treated with sodium hyaluronate-2 exhibits no significant antibacterial effect.

As shown in Table 8, the initial concentration of the pathogenic *Escherichia coli* suspension is $1.64\times10^6$ CFU/mL, the inhibition rate for the control group is 11%, while the inhibition rate of *Escherichia coli* for the cell lysate group treated with sodium hyaluronate-1 is 99%. The results indicate that the vaginal epithelial cell lysate treated with sodium hyaluronate-1 can inhibit the growth of pathogenic *Escherichia coli*, whereas the vaginal epithelial cell lysate treated with sodium hyaluronate-2 exhibits no significant antibacterial effect.

As shown in Table 9, the initial concentration of the pathogenic *Escherichia coli* suspension is $5.9\times10^5$ CFU/mL, the inhibition rate for the control group is 11%, while the inhibition rate of pathogenic *Escherichia coli* for the cell lysate group treated with sodium hyaluronate-1 is 98%. The results show that intestinal epithelial cell lysate treated with sodium hyaluronate-1 inhibits the growth of pathogenic *Escherichia coli*, whereas intestinal epithelial cell lysate treated with sodium hyaluronate-2 exhibits no significant antibacterial effect.

As shown in Table 10, the initial concentration of the *Staphylococcus aureus* suspension is $4.8\times10^5$ CFU/mL, the inhibition rate for the control group is 18%, while the inhibition rate of *Staphylococcus aureus* for the cell lysate group treated with sodium hyaluronate-1 is 97%. The results show that the cell lysate treated with sodium hyaluronate-1 can inhibit the growth of *Staphylococcus aureus,* whereas the cell lysate treated with sodium hyaluronate-2 exhibits no significant antibacterial effect.

As can be seen from Tables 7-10, none of sodium hyaluronate-1 and sodium hyaluronate-2 exhibits the antibacterial effect. However, sodium hyaluronate-1, i.e., hyaluronic acid with an uronic acid terminus, can promote the expression of the cell antimicrobial peptide HBD2, thereby enhancing the innate antibacterial activity of cells. This provides a novel inducer for the production of antimicrobial peptides and also indicates a new direction for clarifying the structure-activity relationship of hyaluronic acid.

The above descriptions are merely embodiments of the present invention, but not intended to limit the present invention. For those skilled in the art, various variations and changes can be made to the present invention. Any amendments, equivalent replacements, improvements and the like made within the spirit and principle of the present invention shall fall within the scope of the appended claims of the present invention.

## Claims

1. Use of hyaluronic acid or a salt thereof in the preparation of an antimicrobial peptide inducer, wherein the reducing end

of the hyaluronic acid or the salt thereof comprises a uronic acid group.

**2.** The use according to claim 1, wherein the molecular weight of the hyaluronic acid or the salt thereof is less than 5000 Da.

**3.** The use according to claim 1, wherein the structural formula of the hyaluronic acid or the salt thereof is represented by Formula (I):

Formula (I)

wherein, X is selected from H, K, Na, Ca or Zn, and n is any integer selected from 0 to 5.

**4.** The use according to claim 1, wherein the antimicrobial peptide comprises human β-defensin 2.

**5.** The use according to claim 1, wherein the hyaluronic acid or the salt thereof is used in regulating human microecology.

**6.** The use according to claim 5, wherein the regulating human microecology comprises inhibiting the growth and proliferation of harmful microbes in the human body.

**7.** The use according to claim 6, wherein the harmful microbes comprise harmful bacteria.

**8.** The use according to claim 7, wherein the harmful bacteria comprise Gram-positive bacteria and/or Gram-negative bacteria.

**9.** The use according to claim 8, wherein the Gram-positive bacteria comprise *Staphylococcus* bacteria; the Gram-negative bacteria comprise *Pseudomonas* bacteria or *Escherichia* bacteria.

**10.** The use according to claim 9, wherein the *Staphylococcus* bacteria comprise *Staphylococcus aureus*; the *Pseudomonas* bacteria comprise *Pseudomonas aeruginosa;* the *Escherichia* bacteria comprise *Escherichia coli*.

**11.** The use according to claim 1, wherein the hyaluronic acid or the salt thereof is used for improving cellular defense capacity.

HBD2

Relative fluorescence intensity (%)

400
300
200
100
0

**
**
ns
ns

Control group
Test group 1
Test group 2
Test group 3
Test group 4

FIG. 1

HBD2
Relative fluorescence intensity (%)
200
150
100
50
0
**
*
ns
ns
Control group 2-0
Test group 2-1
Test group 2-2
Test group 2-3
Test group 2-4

FIG. 2

HBD2
Relative fluorescence intensity (%)
200
150
100
50
0
**
*
ns
ns
Control group 3-0
Test group 3-1
Test group 3-2
Test group 3-3
Test group 3-4

FIG. 3

HBD2
Relative fluorescence intensity (%)
150
100
50
0
*
*
ns
ns
Control group 4-0
Test group 4-1
Test group 4-2
Test group 4-3
Test group 4-4

FIG. 4

**INTERNATIONAL SEARCH REPORT**

International application No.
**PCT/CN2024/134646**

**A. CLASSIFICATION OF SUBJECT MATTER**

A61K31/728(2006.01)i; A61P31/00(2006.01)i

According to International Patent Classification (IPC) or to both national classification and IPC

**B. FIELDS SEARCHED**

Minimum documentation searched (classification system followed by classification symbols)

IPC: A61K 31/-; A61P 31/-

Documentation searched other than minimum documentation to the extent that such documents are included in the fields searched

Electronic data base consulted during the international search (name of data base and, where practicable, search terms used)

CNTXT, ENTXTC, DWPI, CNKI, REGISTRY, CAPLUS, ISI Web of Science: 华熙生物, 透明质酸, 玻尿酸, 糖醛酸, 抗菌肽, 抗菌蛋白, 抗微生物肽, 抗微生物蛋白, 防御素, 防卫素, 还原端, 还原末端, 细菌, 革兰氏阳性菌, 革兰氏阴性菌, 葡萄球菌, 假单胞菌, 埃希氏菌, 大肠杆菌, 9004-61-9, 372146-20-8, hyaluronic, HBD, antibacterial, antimicrobial, peptide, bacteria, gram positive, gram negative, staphylococci, pseudomonas, escherichia

**C. DOCUMENTS CONSIDERED TO BE RELEVANT**

| Category* | Citation of document, with indication, where appropriate, of the relevant passages | Relevant to claim No. |
|---|---|---|
| PX | CN 118059120 A (BLOOMAGE BIOTECHNOLOGY CORPORATION LIMITED) 24 May 2024 (2024-05-24)<br>claims 1-10, and description, paragraph [0022] | 1-11 |
| A | CN 106309471 A (HUI MIZHOU) 11 January 2017 (2017-01-11)<br>abstract, and claim 1 | 1-11 |
| A | US 2014072621 A1 (THE CLEVELAND CLINIC FOUNDATION) 13 March 2014 (2014-03-13)<br>description, paragraph [0006] | 1-11 |
| A | US 2015343014 A1 (LABORATOIRES THEA SAS) 03 December 2015 (2015-12-03)<br>description, paragraphs [0017], [0040] and [0070]-[0094] | 1-11 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 February 2025** | **05 March 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/134646**

**C. DOCUMENTS CONSIDERED TO BE RELEVANT**

| Category* | Citation of document, with indication, where appropriate, of the relevant passages | Relevant to claim No. |
|---|---|---|
| A | HILL, D. R. et al. "Human Milk Hyaluronan Enhances Innate Defense of the Intestinal Epithelium" *Journal of Biological Chemistry,* Vol. 288, No. 40, 04 October 2013 (2013-10-04), 29090-29104 ISSN: 0021-9258, page 29090, abstract | 1-11 |
| A | HILL, D. R. et al. "Specific-sized Hyaluronan Fragments Promote Expression of Human β-Defensin 2 in Intestinal Epithelium" *Journal of Biological Chemistry,* Vol. 287, No. 36, 31 August 2012 (2012-08-31), 30610-30624 ISSN: 0021-9258, page 30610, abstract | 1-11 |
| A | GARIBOLDI, S. et al. "Low Molecular Weight Hyaluronic Acid Increases the Self-Defense of Skin Epithelium by Induction of β-Defensin 2 via TLR2 and TLR4" *Journal of Immunology,* Vol. 181, No. 3, 01 August 2008 (2008-08-01), 2103-2110 ISSN: 0022-1767, page 2103, abstract | 1-11 |

Form PCT/ISA/210 (second sheet) (July 2022)

**INTERNATIONAL SEARCH REPORT**
**Information on patent family members**

International application No.
**PCT/CN2024/134646**

| Patent document cited in search report | Publication date (day/month/year) | Patent family member(s) | Publication date (day/month/year) |
|---|---|---|---|
| CN 118059120 A | 24 May 2024 | CN 117717567 A | 19 March 2024 |
| CN 106309471 A | 11 January 2017 | None | |
| US 2014072621 A1 | 13 March 2014 | US 2018117078 A1 | 03 May 2018 |
| | | US 10653714 B2 | 19 May 2020 |
| | | WO 2012154738 A1 | 15 November 2012 |
| US 2015343014 A1 | 03 December 2015 | PT 2961481 T | 18 August 2017 |
| | | FR 3002452 A1 | 29 August 2014 |
| | | FR 3002452 B1 | 12 February 2016 |
| | | LT 2961481 T | 25 October 2017 |
| | | MA 38344 A1 | 30 March 2018 |
| | | MA 38344 B1 | 30 November 2018 |
| | | CA 2897518 A1 | 04 September 2014 |
| | | CA 2897518 C | 16 February 2021 |
| | | HUE 034470 T2 | 28 February 2018 |
| | | CY 1119431 T1 | 07 March 2018 |
| | | SI 2961481 T1 | 31 January 2018 |
| | | KR 20150134325 A | 01 December 2015 |
| | | KR 102193453 B1 | 21 December 2020 |
| | | JP 2016510017 A | 04 April 2016 |
| | | JP 6440637 B2 | 19 December 2018 |
| | | ES 2641492 T3 | 10 November 2017 |
| | | AR 094976 A1 | 09 September 2015 |
| | | TN 2015000282 A1 | 03 October 2016 |
| | | EP 2961481 A1 | 06 January 2016 |
| | | EP 2961481 B1 | 28 June 2017 |
| | | MX 2015010606 A | 21 April 2016 |
| | | MX 352060 B | 08 November 2017 |
| | | DK 2961481 T3 | 09 October 2017 |
| | | PL 2961481 T3 | 31 January 2018 |
| | | BR 112015020405 A2 | 18 July 2017 |
| | | BR 112015020405 B1 | 07 July 2020 |
| | | WO 2014131974 A1 | 04 September 2014 |
| | | HRP 20171446 T1 | 29 December 2017 |
| | | UA 115894 C2 | 10 January 2018 |
| | | RU 2015140996 A | 06 April 2017 |
| | | RU 2015140996 A3 | 12 March 2018 |
| | | RU 2668827 C2 | 02 October 2018 |
| | | EP 3287171 A1 | 28 February 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202311634295
- CN 202410080031